# EUROPEAN PATENT SPECIFICATION

(11) **EP 1 852 078 B1**
(45) Date of publication and mention of the grant of the patent: **03.11.2010**
(21) Application number: 07009025.3
(22) Date of filing: 04.05.2007
(51) Int. Cl.: A61B 18/14, A61N 5/06

(54) **Combined energy level button**
Kombinierte Energiestufentaste
Bouton de niveau d'énergie combiné

(30) Priority: 05.05.2006 US 418878
(43) Date of publication of application: 07.11.2007
(73) Proprietor: Covidien AG, 8212 Neuhausen am Rheinfall (CH)
(72) Inventor: Guerra, Paul, Boulder CO 80304 (US)
(74) Representative: HOFFMANN EITLE

(56) References cited:
- EP-A- 1 764 057
- WO-A-2005/004734
- US-A- 4 936 842
- US-A1- 2004 092 927

## Description

### BACKGROUND

The present invention relates to a surgical device, preferably an electrosurgical forceps and, more particularly, the present invention relates to a switch on the surgical device that can both adjust electrosurgical energy levels and activate electrosurgical energy.

### Technical Field

During different types of surgery, doctors and surgeons utilize different types of surgical devices. Many of these surgical devices perform several different functions. Each function may be performed by engaging a certain control feature, including a switch, button, trigger, slide or the like, located on the surgical device. Thus, it is not uncommon for a surgical device to include several different control features thereon.
US 2004/0092927, US 4,936,842 and WO 2005/004734 were cited as relevant prior art documents during examination of this patent. The pre-amble of claim 1 is based on US 2004/0092927.

### SUMMARY

The invention is as defined in the appended set of claims.

The present invention relates to a surgical device as defined in claim 1 for use with various surgical procedures. The surgical device (e.g., open-style forceps, in-line-style forceps, or electrosurgical pencil) includes a housing with an activation switch. The activation switch is adapted to connect to an electrosurgical energy source and includes a knob. The knob is slideable within a guide channel within the housing and the knob may be biased in an inactivated position. The activation switch is selectively moveable in a first direction within the guide channel to set a desired level of electrosurgical energy. The activation switch is also selectively moveable in a second direction to activate the electrosurgical energy source and is designed and configured to set the intensity level of electrosurgical energy before the activation of electrosurgical energy.

The activation switch is configured to electromechanically cooperate with a voltage divider network to adjust or control the intensity or energy levels of the surgical device.

The guide channel is dimensioned to include a plurality of discreet positions. In such an embodiment, the knob is slideable within the guide channel between the plurality of discreet positions. In an embodiment, tactile feedback is provided to a user when the knob is slid between the plurality of discreet positions.

The present disclosure also relates to an electrosurgical system that utilizes the disclosed surgical device. Depressing the knob may activate electrosurgical energy and sliding the knob along the guide channel may set the intensity of electrosurgical energy.

In another embodiment according to the present disclosure, the knob may be biased towards a first depressible position where it does not activate electrosurgical energy. Depressing the knob into a second depressible position activates electrosurgical energy and releasing the knob will cause the knob to return to its first depressible position, thus deactivating electrosurgical energy.

The present invention also relates to an electrosurgical system for performing electrosurgery on a patient and includes an electrosurgical generator which provides electrosurgical energy to the surgical device. The surgical device includes an active electrode that supplies electrosurgical energy to a patient and an electrosurgical return electrode that returns the electrosurgical energy to the electrosurgical generator.

For a better understanding of the present disclosure and to show how it may be carried into effect, reference is now made by way of example to the accompanying drawings.

### BRIEF DESCRIPTION OF THE DRAWINGS

Various embodiments of the present disclosure are described herein with reference to the drawings wherein:

FIG. 1 is a perspective view of an endoscopic forceps comprising an activation switch according to one embodiment of the present disclosure;

FIG. 2 is a top view of the endoscopic forceps of FIG. 1;

FIG. 3 is a side view of the endoscopic forceps of FIG. 1;

FIG. 4 is an enlarged side view of the activation switch illustrated on an endoscopic forceps;

FIG. 5A is a schematic, cross-sectional view of the activation switch in an inactivated position;

FIG. 5B is a schematic, cross-sectional view of the activation switch in an activated position;

FIG. 6 is a perspective view of an open-style forceps having an activation switch;

FIG. 7 is a perspective view an electrosurgical pencil with parts separated having an activation switch; and

FIG. 8 is a perspective view of an in-line-style forceps having an activation switch.

### DETAILED DESCRIPTION

Embodiments of the presently disclosed activation switch and method of using the same (a method does not form an embodiment of the present invention) are described below with reference to the accompanying figures wherein like reference numerals identify similar or identical elements. In the following description, well-known functions or constructions are not described in detail to avoid obscuring the disclosure in unnecessary detail. As used herein and as is traditional, the term "distal" refers to that portion that is farthest from the user while the term "proximal" refers to that portion that is closest to the user.

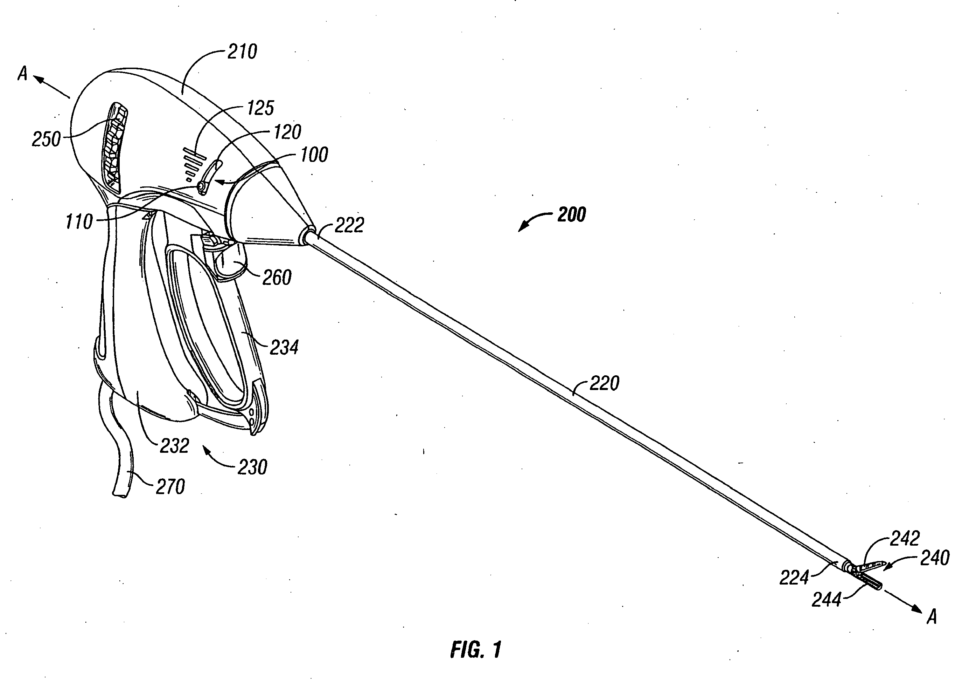

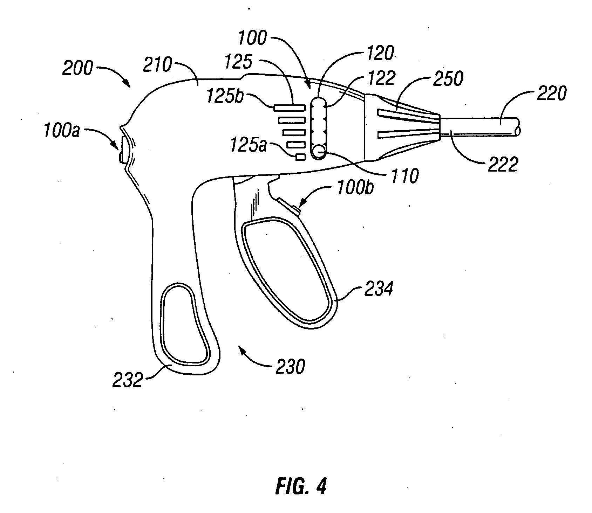

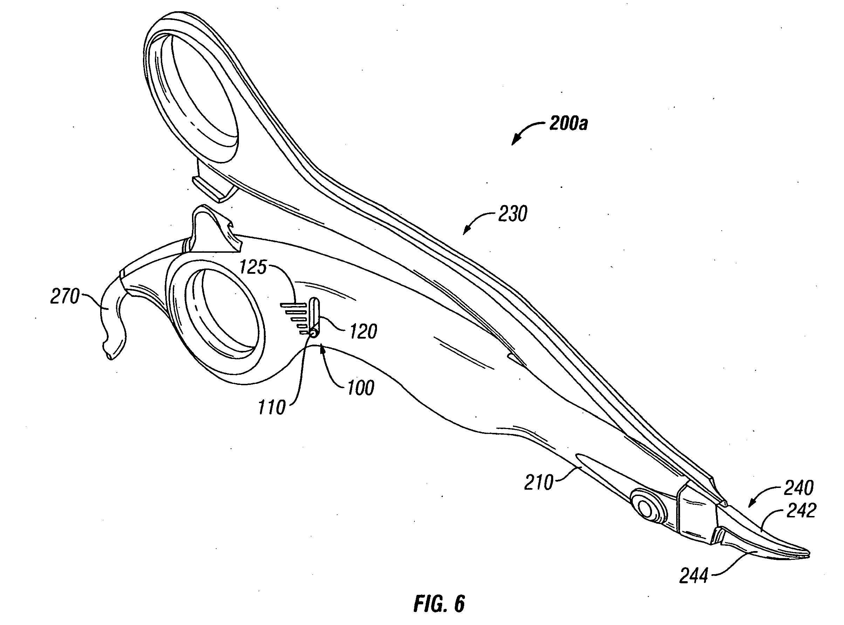

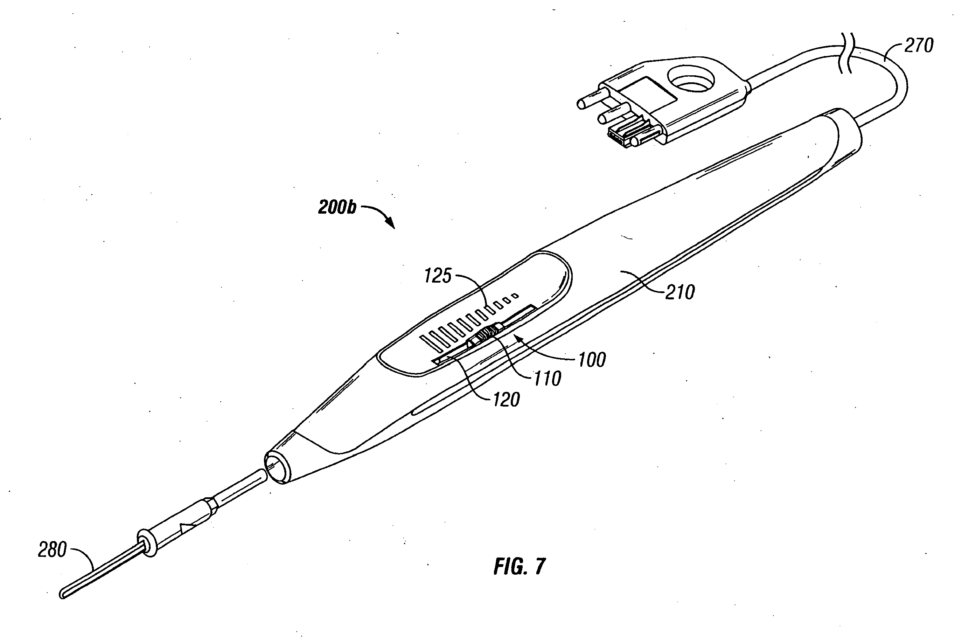

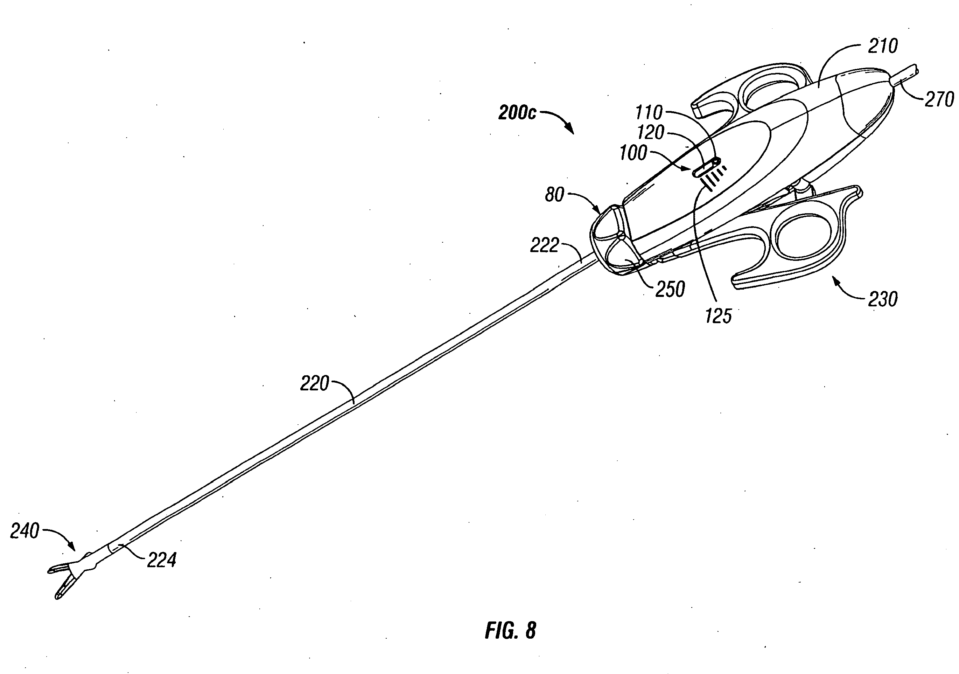

In general, the various figures illustrate an activation switch 100 disposed on a variety of different surgical devices. Specifically, FIGS. 1-4 illustrate the activation switch 100 on an endoscopic forceps 200; FIG. 6 illustrates the activation switch 100 on an open-style forceps 200a; FIG. 7 illustrates the activation switch 100 on an electrosurgical pencil 200b; and FIG. 8 illustrates the activation switch 100 on an in-line-style forceps 200c. Other suitable types of surgical devices, which are not shown, may include the activation switch 100 envisioned herein. The activation switch 100 may be configured to activate a monopolar energy mode, a bipolar energy mode or a combination thereof. As can be appreciated, one or more activation switches 100 can be disposed on a surgical device 200 (for instance, on the housing 210 and/or on the handle assembly 230) for activating a different type of energy, e.g., three activation switches 100, 100a and 100b are illustrated in FIG. 4.

Initially referring to FIGS. 1-4 and 6-8, illustrations of an endoscopic surgical device including the activation switch 100 are shown and are generally referred to by reference numeral 200. Surgical device 200 may include a housing 210, a shaft 220 defining axis "A-A," activation switch 100, an end effector assembly 240, a handle assembly 230, a rotation assembly 250 and a trigger assembly 260.

As best illustrated in FIG. 4, the activation switch 100 is disposed at least partially on the housing 210 and includes a knob 110 and a guide channel 120. Knob 110 of the activation switch 100 is slidingly supported in the guide channel 120 and is operable to both activate electrosurgical energy and to set the intensity of energy levels of electrosurgical energy in surgical devices 200. For example, sliding the knob 110 along the guide channel 120 sets the intensity of the desired electrosurgical energy and depressing or otherwise moving the knob 110 relative to or along the housing activates the electrosurgical energy. In an exemplary embodiment as illustrated in FIGS. 1-4, the knob 110 is biased towards a first inactive position. Depressing knob 110 into a second depressible position (i.e., inwardly relative to the housing) activates electrosurgical energy. Releasing knob 110 will cause knob 110 to return to about the first inactive position. Indicia 125 may be included on the surgical device 200 that corresponds to an intensity level of electrosurgical energy when the knob 110 is activated.

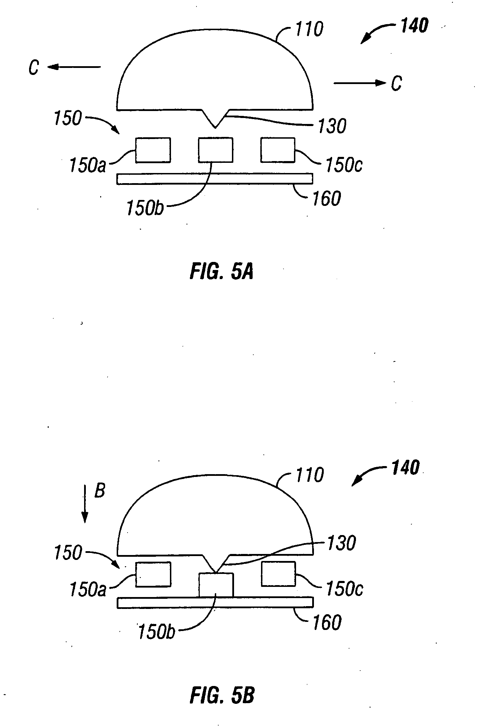

With reference to FIGS. 5A and 5B, details of one embodiment of the operation of the activation switch 100 are described with reference to FIGS. 5A and 5B. Knob 110 includes a protrusion 130 that depends from a bottom surface thereof. The protrusion 130 is configured to selectively contact a voltage divider network 140 (hereinafter referred to as "VDN") upon movement of knob 110 relative to the housing 210 (see arrow "B"). The VDN 140 includes a plurality of traces 150 disposed atop a base or substrate 160. When the knob 110 is selectively positioned in the guide channel 120 (along arrow "C"), the knob 110 is depressed to activate the electrosurgical energy. More particularly, and as best shown in FIG. 5B, depression of knob 110 engages one of the plurality of traces 150 (in this case trace 150b) to activate the instrument with a particular electrosurgical intensity. For example, when trace 150b is engaged and contacts a portion of the substrate 160 (illustrated in FIG. 5B), electrosurgical energy is activated. Further, the intensity of electrosurgical energy depends on where within the guide channel 120 the knob 110 is positioned, which corresponds to one of the plurality of traces 150. The VDN 140 may be electrically connected to a source of electrosurgical energy and it may control the intensity of electrosurgical energy.

The activation switch 100 may function as a slide potentiometer, sliding over and along VDN 140. In an exemplary embodiment shown in FIG. 4, a momentary switch is coupled to the sliding potentiometer. The activation switch 100 has a first position wherein the knob 110 is at a proximal-most position (closest to smallest indicia 125a) corresponding to a zero intensity setting, a second position wherein the knob 110 is at a distal-most position (closest to largest indicia 125b) corresponding to a relative high intensity setting, and a plurality of intermediate positions wherein the knob 110 is positioned between the distal-most position and the proximal-most position corresponding to various intermediate intensity settings. As can be appreciated, the intensity settings from the proximal end to the distal end may be reversed.

With continued reference to FIG. 4, the guide channel 120 is provided with a series of cooperating discreet or detented positions 122 defining a series of positions to allow easy selection of the output intensity from the low intensity setting to the high intensity setting. These positions 122 are illustrated in FIG. 4 on the guide channel 120. In an alternative that is not an embodiment of the invention, it is also envisioned that the knob 110 includes positions 122. In an exemplary embodiment, the positions 122 enable the knob 110 to snap into position with the guide channel 120 at positions where the knob 110 aligns with traces 150.

The series of cooperating discreet or detented positions 122 may provide a surgeon with a degree of tactile feedback. Accordingly, in use, as the knob 110 slides distally and proximally, tactile feedback may be provided to the user to inform him of when the knob 110 has been set to the desired intensity setting. A visual level of tactile feedback may be incorporated into activation switch 100. As such, the knob 110 may move a colored component (not explicitly shown) under housing 210 that would be visible through openings (not explicitly shown) in housing 210. Each opening may correspond to a particular energy level or trace 150. It is also envisioned for the positions 122 (or another feature of endoscopic forceps 200) or the generator to provide audible feedback.

The activation switch 100 may be operable to adjust the power parameters (e.g., voltage, power and/or current intensity) and/or the power verses impedance curve shape to affect the perceived output intensity. For example, and with particular respect to the electrosurgical pencil shown in FIG. 7, the greater the knob 110 is displaced in a distal direction, the greater the level of power parameters transmitted to the end effector assembly 240. It is envisioned for the current intensities to be in the range of about 60 mA to about 240 mA when using an end effector assembly 240 and having a typical tissue impedance of about 2K ohms. An intensity level of 60 mA provides light and/or minimal cutting/dissecting/hemostatic effects, while an intensity level of 240 mA would provide aggressive cutting/dissecting/hemostatic effects. Accordingly, the range of current intensity may be from about 100 mA to about 200 mA at 2K ohms.

The intensity settings may be preset and selected from a look-up table based on a choice of electrosurgical instruments/attachments, desired surgical effect, surgical specialty and/or surgeon preference. The selection may be made automatically or selected manually by the user.

In operation, and depending on the particular electrosurgical function desired, the surgeon moves the knob 110 to a desired level and depresses the knob 110, which depresses one of the corresponding traces 150a-150c (see FIGS. 5A and 5B) into contact with the pad 160, thereby transmitting a respective characteristic signal or voltage level to an electrosurgical generator. For example, the surgeon can depress trace 150a to perform a cutting and/or dissecting function, trace 150b to perform a blending function, or trace 150c to perform a hemostatic function. In turn, a generator transmits an appropriate waveform output to the end effector assembly 240.

To vary the intensity of the power parameters of the surgical device 200, the surgeon moves the knob 110. As mentioned above, in one embodiment, the intensity may be varied from about 60 mA for a light effect to about 240 mA for a more aggressive effect. When the knob 110 of the activation switch 100 is positioned at the proximal-most end of the guide channel 120, the VDN 140 is set to a null and open position, corresponding to an intensity level of zero.

An RF line (not explicitly shown) for transmitting RF energy to an electrode may be provided and may be directly electrically connected to an electrode receptacle. In such an embodiment, since RF line is directly connected to electrode receptacle, RF line bypasses VDN 140 and thus isolates VDN 140. Such an arrangement may reduce the risk of the VDN 140 becoming overheated.

With specific reference to FIG. 4, an enlarged view of the activation switch 100 is shown depicted on the endoscopic forceps 200. As shown in FIG. 4, the activation switch 100 may be located on at least one of a variety of suitable positions on the endoscopic forceps 200. In the embodiment of FIG. 4, activation switch 100 is illustrated in three different locations: housing 210, fixed handle 232 and movable handle 234.

Additional elements of the surgical device 200 are discussed with reference to the endoscopic forceps 200 of FIGS. 1-4. As can be appreciated, the surgical devices illustrated in the remaining figures may also be used with the activation switch 100 and are a part of this disclosure. As mentioned above and as shown in FIG. 4, the surgical device 200 may include housing 210, shaft 220, activation switch 100, end effector assembly 240, handle assembly 230, rotation assembly 250 and trigger 260. Handle assembly 230 of the endoscopic forceps 200 includes a fixed handle 232 and a movable handle 234. The fixed handle 232 is integrally associated with the housing 210 and the movable handle 234 is movable relative to the fixed handle 232. The movable handle 234 may be coupled to the housing 210 and to the fixed handle 232. Additionally, the handle assembly 230 may include a pair of upper flanges that cooperate with the handle assembly 230 to actuate the drive assembly. More particularly, the upper flange may also include a force-actuating flange or drive flange, which abuts the drive assembly such that pivotal movement of the moveable handle 234 forces the actuating flange against the drive assembly which, in turn, closes the jaw members 242 and 244.

Rotation assembly 250 may be integrally associated with the housing 210 and may be rotatable approximately 180 degrees in either direction about the axis "A-A." The rotation assembly 250 may be located at one of a plurality of locations on the housing 210. An example of two such locations are illustrated in FIGS. 1 and 4.

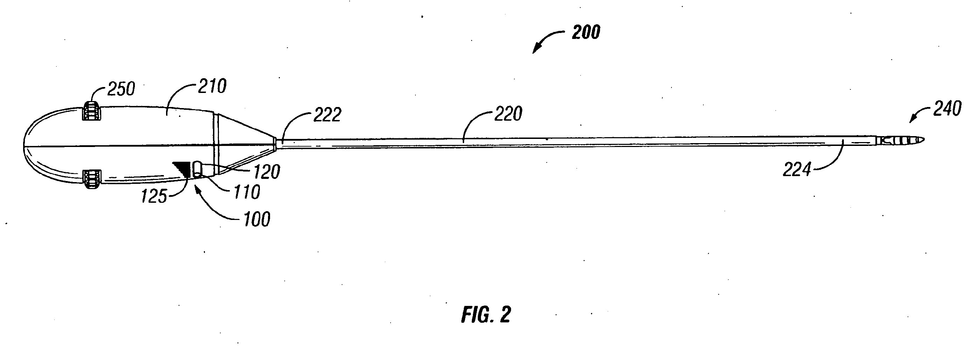

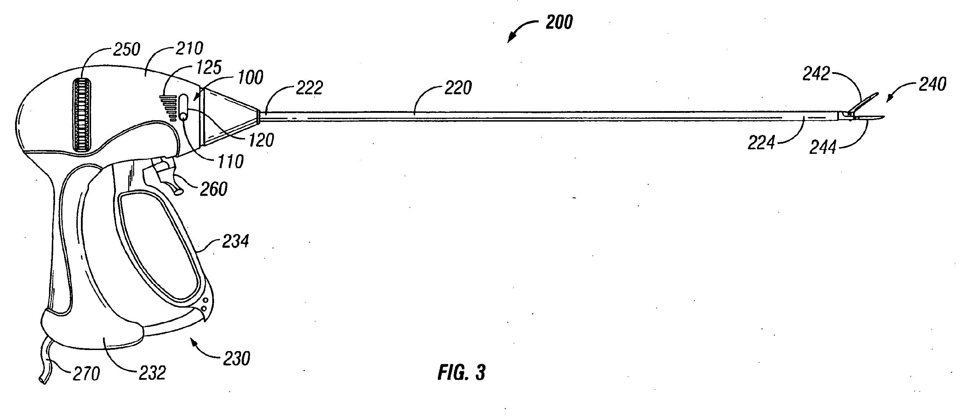

A proximal end 222 of the shaft 220 is in mechanical cooperation with the housing 210. The end effector assembly 240 is attached at a distal end 224 of the shaft 220 and includes a pair of opposing jaw members 242 and 244. The movable handle 234 of the handle assembly 230 is ultimately connected to a drive assembly which, together, mechanically cooperate to impart movement of the jaw members 242 and 244 from an open position wherein the jaw members 242 and 244 are disposed in spaced relation relative to one another (FIGS. 1 and 3), to a clamping or closed position (FIG. 2) wherein the jaw members 242 and 244 cooperate to be able to grasp tissue therebetween. When the jaw members 242 and 244 are fully compressed about tissue, the endoscopic forceps 200 is ready for selective application of electrosurgical energy and subsequent separation of the tissue. More particularly, as energy is being selectively transferred to the end effector assembly 240, across the jaw members 242 and 244 and through the tissue, a tissue seal forms isolating two tissue halves. At this point, the user may cut the tissue seal via the trigger assembly 260.

As shown in FIGS. 1 and 3, the endoscopic forceps 200 may also include an electrosurgical cable 270 that connects the endoscopic forceps 200 to a source of electrosurgical energy, e.g., a generator (not explicitly shown). Generators such as those sold by Valleylab - a division of Tyco Healthcare LP, located in Boulder Colorado may be used as a source of electrosurgical energy, e.g., FORCE EZ^{™} Electrosurgical Generator, FORCE FX^{™} Electrosurgical Generator, FORCE 1C^{™}, FORCE 2^{™} Generator, SurgiStat^{™} II.

The generator may include various safety and performance features including isolated output and independent activation of accessories. The electrosurgical generator may include Valleylab's Instant Response^{™} technology features which provide an advanced feedback system to sense changes in tissue 200 times per second and adjust voltage and current to maintain appropriate power. The Instant Response^{™} technology is believed to provide one or more of the following benefits to surgical procedure:
- Consistent clinical effect through all tissue types;
- Reduced thermal spread and risk of collateral tissue damage;
- Less need to "turn up the generator"; and
- Designed for the minimally invasive environment.

The electrosurgical cable 270 may be internally divided into cable leads which each transmit electrosurgical energy through their respective feed paths through the endoscopic forceps 200 to the end effector assembly 240. The housing 210, the rotation assembly 250, the activation switch 100, the handle assembly 230, the trigger assembly 260 and their respective inter-cooperating component parts along with the shaft 220 and the end effector assembly 240 may all be assembled during the manufacturing process to form a partially and/or fully disposable endoscopic forceps 200. For example, the shaft 220 and/or the end effector assembly 240 may be disposable and, therefore, selectively/releasably engagable with the housing 210 and the rotation assembly 250 to form a partially disposable endoscopic forceps 200 and/or the entire endoscopic forceps 200 may be disposable after use.

The method of the present disclosure (a method does not form an embodiment of the present invention) includes using the surgical device 200 to administer electrosurgical energy to a patient. The method includes the steps of providing a surgical device 200 including an activation switch 100, as described above, sliding the knob 110 within the guide channel 120 to set the intensity of electrosurgical energy, and depressing the knob 110 to activate electrosurgical energy.

The present disclosure also includes an electrosurgical system for performing electrosurgery on a patient. The electrosurgical system includes an electrosurgical generator that provides electrosurgical energy, an active electrode that supplies energy to a patient, an electrosurgical return electrode that returns electrosurgical energy to the electrosurgical generator, and the surgical device 200 having an activation switch 100, as described above.

While several embodiments of the disclosure are shown in the drawings, it is not intended that the disclosure be limited thereto, as it is intended that the disclosure be as broad in scope as the art will allow and that the specification be read likewise. Therefore, the above description should not be construed as limiting, but merely as exemplifications of various embodiments. Those skilled in the art will envision other modifications within the scope of the claims appended hereto.

## Claims

1. A surgical device (200, 200a, 200b, 200c), comprising:
a housing (210) having an activation switch (100) disposed thereon, the activation switch adapted to couple to an electrosurgical energy source, the activation switch including a knob (110) slidingly disposed within a guide channel (120) defined within the housing wherein the guide channel comprises a plurality of discreet positions (122), the knob being slideable between the plurality of discreet positions; and
the activation switch being selectively moveable in a first direction within the guide channel to set a desired electrosurgical energy level, wherein the activation switch is selectively moveable in a second direction to activate the electrosurgical energy source, and is operable to set the intensity level of electrosurgical energy before electrosurgical energy is activated; wherein
the activation switch electromechanically cooperates with a voltage divider network (140) to adjust energy levels, **characterized in that**:
the knob includes a protrusion (130) that depends from a bottom surface thereof,
the protrusion is configured to selectively contact the voltage divider network (140) upon movement of the knob relative to the housing,
the voltage divider network includes a plurality of traces (150) disposed atop a base or substrate (160), and
the intensity of electrosurgical energy depends on where within the guide channel the knob is positioned, which corresponds to one of the plurality of traces, such that:
when the knob is selectively positioned in the guide channel, the knob is depressible to activate the electrosurgical energy, and depression of the knob engages one of the plurality of traces to activate the instrument with a particular electrosurgical intensity, whereby when one of the traces is engaged and contacts a portion of the substrate, electrosurgical energy is activated.

2. The surgical device according to claim 1, wherein the knob is biased in an inactivated position.

3. The surgical device according to claim 2, wherein the knob is depressible to a depressed position so as to activate the electrosurgical energy and the bias serves to return the knob to its inactivated position upon release thereof to deactivate the electrosurgical energy.

4. The surgical device according to any one of the preceding claims, wherein tactile feedback is provided to a user when the knob is slid between the plurality of discreet positions on the guide channel.

5. The surgical device according to any preceding claim, wherein the device is an electrosurgical pencil (200b).

6. The surgical device according to any one of claims 1 to 5, wherein the device is an open-style forceps (200a).

7. The surgical device according to any one of claims 1 to 5, wherein the device in an in-line-style forceps (200c).

8. The surgical device according to any one of the preceding claims configured so that depressing the knob activates electrosurgical energy and sliding the knob along the guide channel sets the intensity level of electrosurgical energy.

9. An electrosurgical system for performing electrosurgery on a patient, the electrosurgical system comprising:
an electrosurgical energy source that provides electrosurgical energy;
an active electrode which supplies electrosurgical energy to a patient;
an electrosurgical return electrode which returns electrosurgical energy to the electrosurgical energy source; and
the surgical device of any one of the preceding claims.

## Patentansprüche

1. Chirurgische Vorrichtung (200, 200a, 200b, 200c), umfassend:
ein Gehäuse (210) mit einem Aktivierungsschalter (100), der darauf angeordnet ist, wobei der Aktivierungsschalter dazu angepasst ist, eine elektrochirurgische Energiequelle zu koppeln, wobei der Aktivierungsschalter einen Knopf (110) enthält, der verschiebbar innerhalb eines Führungskanals (120), der innerhalb des Gehäuses definiert ist, angeordnet ist, wobei der Führungskanal eine Mehrzahl diskreter Positionen (122) aufweist, wobei der Knopf zwischen der Mehrzahl diskreter Positionen verschiebbar ist; und
wobei der Aktivierungsschalter wahlweise in einer ersten Richtung innerhalb des Führungskanals beweglich ist, um ein gewünschtes elektrochirurgisches Energieniveau einzustellen, wobei der Aktivierungsschalter wahlweise in eine zweite Richtung beweglich ist, um die elektrochirurgische Energiequelle zu aktivieren, und dazu betrieben werden kann, das Intensitätsniveau elektrochirurgischer Energie einzustellen, bevor elektrochirurgische Energie aktiviert wird;
wobei der Aktivierungsschalter elektromechanisch mit einem Spannungsteilernetzwerk (140) zusammenwirkt, um Energieniveaus einzustellen, **dadurch gekennzeichnet, dass**:
der Knopf einen Vorsprung (130) enthält, der von einer unteren Oberfläche von ihm absteht,
wobei der Vorsprung dazu ausgestaltet ist, wahlweise das Spannungsteilernetzwerk (140) nach einer Bewegung des Knopfs relativ zu dem Gehäuse zu kontaktieren,
wobei das Spannungsteilernetzwerk eine Mehrzahl von Spuren (150) enthält, die auf einer Basis oder einem Substrat (160) angeordnet sind, und
die Intensität elektrochirurgischer Energie davon abhängt, wo der Knopf in dem Führungskanal positioniert ist, was einer der Mehrzahl von Spuren entspricht, so dass:
wenn der Knopf wahlweise in dem Führungskanal positioniert ist, der Knopf eingedrückt werden kann, um die elektrochirurgische Energie zu aktivieren, und das Eindrücken des Knopfs eine der Mehrzahl von Spuren einklinkt, um das Instrument mit einer bestimmten elektrochirurgischen Intensität zu aktivieren, wodurch, wenn eine der Spuren eingeklinkt ist und einen Teil des Substrats kontaktiert, elektrochirurgische Energie aktiviert wird.

2. Chirurgische Vorrichtung nach Anspruch 1, wobei der Knopf in eine nicht aktivierte Position vorgespannt ist.

3. Chirurgische Vorrichtung nach Anspruch 2, wobei der Knopf in eine eingedrückte Position eingedrückt werden kann, um die elektrochirurgische Energie zu aktivieren, und die Vorspannung dazu dient, den Knopf nach seinem Lösen in seine nicht aktivierte Position zurückzuführen, um die elektrochirurgische Energie zu deaktivieren.

4. Chirurgische Vorrichtung nach einem der vorhergehenden Ansprüche, wobei eine taktile Rückmeldung für einen Benutzer bereitgestellt wird, wenn der Knopf zwischen der Mehrzahl diskreter Positionen an dem Führungskanal verschoben wird.

5. Chirurgische Vorrichtung nach einem vorhergehenden Anspruch, wobei die Vorrichtung ein elektrochirurgischer Stift (200b) ist.

6. Chirurgische Vorrichtung nach einem der Ansprüche 1 bis 5, wobei die Vorrichtung ein offener Forceps (200a) ist.

7. Chirurgische Vorrichtung nach einem der Ansprüche 1 bis 5, wobei die Vorrichtung ein Inline-Forceps (200c) ist.

8. Chirurgische Vorrichtung nach einem der vorhergehenden Ansprüche, die so ausgestaltet ist, dass ein Eindrücken des Knopfs elektrochirurgische Energie aktiviert und ein Verschieben des Knopfes entlang des Führungskanals das Intensitätsniveau elektrochirurgischer Energie einstellt.

9. Elektrochirurgisches System zum Durchführen von Elektrochirurgie an einem Patienten, wobei das elektrochirurgische System umfasst:
eine elektrochirurgische Energiequelle, die elektrochirurgische Energie bereitstellt;
eine aktive Elektrode, die einem Patienten elektrochirurgische Energie zuführt;
eine elektrochirurgische Rückführelektrode, die elektrochirurgische Energie zu der elektrochirurgischen Energiequelle zurückführt; und
die chirurgische Vorrichtung nach einem der vorhergehenden Ansprüche.

## Revendications

1. Dispositif chirurgical (200, 200a, 200b, 200c), comprenant:
un boîtier (210) ayant un commutateur d'activation (100) disposé sur celui-ci, le commutateur d'activation étant apte à être couplé à une source d'énergie électrochirurgicale, le commutateur d'activation comportant un bouton (110) disposé d'une manière coulissante dans un canal de guidage (120) défini dans le boîtier, où le canal de guidage comprend plusieurs positions discrètes (122), le bouton pouvant coulisser entre la pluralité de positions discrètes; et le commutateur d'activation étant sélectivement déplaçable dans une première direction dans le canal de guidage pour établir un niveau d'énergie électrochirurgicale souhaité, où le commutateur d'activation est déplaçable sélectivement dans une seconde direction pour activer la source d'énergie électrochirurgicale et est actionnable pour établir le niveau d'intensité de l'énergie électrochirurgicale avant que l'énergie électrochirurgicale ne soit activée; où
le commutateur d'activation coopère électromécaniquement avec un réseau de division de tension (140) pour ajuster les niveaux d'énergie, **caractérisé en ce que**:
le bouton comporte une saillie (130) qui s'étend vers le bas depuis une surface inférieure de celui-ci,
la saillie est configurée pour venir sélectivement en contact avec le réseau de division de tension (140) lors d'un déplacement du bouton relativement au boîtier,
le réseau de division de tension comporte une pluralité de traces (150) disposées sur une base ou un substrat (160), et
l'intensité de l'énergie électrochirurgicale dépend de l'endroit dans le canal de guidage où le bouton est positionné, qui correspond à une d'une pluralité de traces, de telle sorte que:
lorsque le bouton est sélectivement positionné dans le canal de guidage, le bouton peut être enfoncé pour activer l'énergie électrochirurgicale, et l'enfoncement du bouton met en prise une de la pluralité de traces pour activer l'instrument avec une intensité électrochirurgicale particulière, par quoi, lorsqu'une des traces est engagée et vient en contact avec une portion du substrat, l'énergie électrochirurgicale est activée.

2. Dispositif chirurgical selon la revendication 1, dans lequel le bouton est sollicité dans une position inactivée.

3. Dispositif chirurgical selon la revendication 2, dans lequel le bouton peut être enfoncé à une position enfoncée de manière à activer l'énergie électrochirurgicale, et la sollication sert à ramener le bouton à sa position inactivée lors de sa libération pour désactiver l'énergie électrochirurgicale.

4. Dispositif chirurgical selon l'une quelconque des revendications précédentes, dans lequel une réaction tactile est fournie à un utilisateur lorsque le bouton est amené à glisser entre la pluralité de positions discrètes sur le canal de guidage.

5. Dispositif chirurgical selon l'une quelconque des revendications précédentes, où le dispositif est un électrocautère (200b).

6. Dispositif chirurgical selon l'une quelconque des revendications 1 à 5, où le dispositif est un forceps de style ouvert (200a).

7. Dispositif chirurgical selon l'une quelconque des revendications 1 à 5, où le dispositif est un forceps du style en ligne (200c).

8. Dispositif chirurgical selon l'une quelconque des revendications précédentes, configuré de façon que l'enfoncement du bouton active l'énergie électrochirurgicale, et le coulissement du bouton le long du canal de guidage établit le niveau d'intensité de l'énergie électrochirurgicale.

9. Système électrochirurgical pour exécuter l'électrochirurgie sur un patient, le système électrochirurgical comprenant:
une source d'énergie électrochirurgicale qui fournit de l'énergie électrochirurgicale;
une électrode active qui fournit l'énergie électrochirurgicale à un patient;
une électrode de retour électrochirurgicale qui ramène l'énergie électrochirurgicale à la source d'énergie électrochirurgicale; et
le dispositif chirurgical selon l'une quelconque des revendications précédentes.
